# EUROPÄISCHE PATENTSCHRIFT

(11) **EP 2 220 482 B1**
(45) Veröffentlichungstag und Bekanntmachung des Hinweises auf die Patenterteilung: **20.03.2013**
(21) Anmeldenummer: 08859335.5
(22) Anmeldetag: 27.11.2008
(51) Int. Cl.: G01N 27/12

(54) **GASSENSOR MIT EINER VERBESSERTEN SELEKTIVITÄT**
GAS SENSOR WITH IMPROVED SELECTIVITY
DÉTECTEUR DE GAZ À SÉLECTIVITÉ AMÉLIORÉE

(30) Priorität: 10.12.2007 DE 102007059652
(43) Veröffentlichungstag der Anmeldung: 25.08.2010
(73) Patentinhaber: EADS Deutschland GmbH, 85521 Ottobrunn (DE)
(72) Erfinder: HELWIG, Andreas, 80469 München (DE); MÜLLER, Gerhard, 85567 Grafing (DE); SPANNHAKE, Jan, 86567 Hilgertshausen-Tandern (DE)
(74) Vertreter: Schäfer, Matthias W.
(86) Internationale Anmeldenummer: PCT/EP2008/010097
(87) Internationale Veröffentlichungsnummer: WO 2009/074232

(56) Entgegenhaltungen:
- WO-A-2007/073111
- DE-A1- 4 401 885
- DE-A1- 19 924 083
- HELWIG ET AL: "Temperature characterization of silicon substrates for gas sensors by Raman spectroscopy" SENSORS AND ACTUATORS B, ELSEVIER SEQUOIA S.A., LAUSANNE, CH, Bd. 126, Nr. 1, 20. September 2007 (2007-09-20), Seiten 240-244, XP022261815 ISSN: 0925-4005
- SPANNHAKE ET AL: "SnO2:Sb - A new material for high-temperature MEMS heater applications: Performance and limitations" SENSORS AND ACTUATORS B, ELSEVIER SEQUOIA S.A., LAUSANNE, CH, Bd. 124, Nr. 2, 7. Juni 2007 (2007-06-07), Seiten 421-428, XP022107828 ISSN: 0925-4005

## Beschreibung

Die vorliegende Erfindung betrifft einen Gassensor zur Detektion von Gasen mit zumindest einer auf einem Substrat aufgebrachten gassensitiven Schicht, wobei weiterhin wenigstens eine Leiterbahn zur Kontaktierung der Schicht auf dem Substrat vorgesehen ist.

Zur Detektion von unterschiedlichen Gasen und Dämpfen werden gegenwärtig kostengünstige Dickschichtmetalloxidsensoren verwendet. Um den Leistungsverbrauch dieser Sensoren zu reduzieren, ist ein aktueller Trend der Entwicklung in Richtung der Dünnfilmtechnologie feststellbar. Die sensitiven Schichten der Gassensoren weisen hierfür eine nominelle Schichtdicke von wenigen 100nm bis zu einzelnen Nanoröhrchen oder Nanoclustem mit Größenordnungen von wenigen nm auf. Weiterhin kann Energie gespart werden, indem diese dünnen Strukturen und Schichten auf mikromechanische Bauteile aufgebracht werden. Gegenüber konventionellen Sensoren ergibt sich hierdurch bei geeignetem Betrieb ein um den Faktor von bis zu 500 geringeren Leistungsverbrauch.

Die auf den Substraten aufgebrachten gassensitiven Schichten werden zumeist im resistiven Messverfahren betrieben, sodass die Änderung des elektrischen Widerstands der gassensitiven Schicht eine Information über das Vorhandensein, die Konzentration und die Art eines zu detektierenden Gases bereitstellt. Da Gassensoren meist bei hohen Temperaturen betrieben werden, wird zur Kontaktierung der sensitiven Schichten ein Edelmetall verwendet.

Ein großer Nachteil der Metalloxidsensoren ist dabei die hohe Reaktivität auf unterschiedliche Gase. Da diese Sensoren sehr stark auf eine Vielzahl von Gasen reagieren, ist eine eindeutige Bestimmung eines Gases und seiner Konzentration insbesondere vor dem Hintergrund verschiedener Temperaturen und der damit verknüpften Temperaturabhängigkeit der gassensitiven Schichten möglich, um eine hinreichend genaue Bestimmung eines Gases und seiner Konzentration zu liefern. Häufig ist aber aufgrund der Temperaturabhängigkeit eine schnelle und genaue Bestimmung der zu messenden Größen nicht mehr möglich.

Folglich werden Sensorarrays aus mehreren Sensoreinheiten verwendet, die aus unterschiedlichen Gassensoren aufgebaut sind, um eine genaue Bestimmung zu erzielen. Die Auswertung wird dabei umso leichter, je unterschiedlicher die Sensoren auf verschiedene Gase reagieren. Negativ beeinflusst wird jedoch das sensitive Verhalten der einzelnen Schichten dadurch, dass Edelmetalle ebenfalls als Katalysatoren fungieren und die chemische Reaktion an der Sensoroberfläche beeinflussen. Das charakteristisch sensitive Verhalten der einzelnen Schichten wird somit vom Katalysator verändert. Sensorschichten, die aufwändig gezüchtet wurden, um unterscheidend zu reagieren, verhalten sich durch die Wirkung des Katalysators wieder annähernd gleich. Dadurch ist die Selektivität vorhandener Gassensoren stark beeinträchtigt.

Da diese Sensoren sehr stark auf eine Vielzahl von Gasen reagieren, ist eine eindeutige Bestimmung nur beim Betrieb der Sensoren bei stark unterschiedlichen Temperaturen gegeben. Daher kann nur die Anwendung weiterer Maßnahmen zu einer Veränderung der sensitiven Eigenschaften von Metalloxidgassensoren führen. Der Betrieb bei unterschiedlichen Temperaturen bietet sich insbesondere beim Einsatz von Sensorarrays an, um unterschiedliche Sensitivitäten und Selektivitäten der einzelnen Komponenten des Arrays zu nutzen. Bei verschiedenen Temperaturstufen werden selektiv verschiedene Sensoreinheiten aktiviert, wobei diese Methode mit dem Nachteil behaftet ist, dass die unterschiedlichen Temperaturstufen erst erreicht werden müssen, wodurch die Reaktionszeit herabgesetzt wird.

Die weiteren Maßnahmen betreffen sensitive Schichten, die mit einem Katalysator dotiert sind (Platin, Gold, Silber, Palladium), wobei als sensitive Schichten Stoffgemenge wie beispielsweise InSe, InSnO verwendet werden. Auch eine Veränderung der Morphologie der sensitiven Schichten, welche glatte, raue oder poröse Oberflächen umfassen können, kann zu einer Erhöhung der Selektivität führen. Neben der oben bereits genannten Anwendung der Sensoren bei unterschiedlichen Temperaturen kann die Aufbringung einer Filterschicht zur Erhöhung der Selektivität beitragen.

Metalloxidgassensoren werden im resistiven Sensorbetrieb betrieben, d.h. die Veränderung ihres elektrischen Widerstands dient als Sensorfunktion. Zum Auslesen des Sensorwiderstandes werden elektrische Kontakte wie die eingangs genannten Leiterbahnen zur Kontaktierung der sensitiven Schichten benötigt. Da Metalloxidgassensoren bei Temperaturen zwischen 300°C und 450°C betrieben werden, werden aus Gründen der Stabilität und nachträglicher Oxidation diese elektrischen Leiterbahnen zur Kontaktierung meist aus Edelmetallen wie Platin oder Gold hergestellt.

Aus der europäischen Patentschrift EP 0 899 563 B1 ist ein gattungsgemäßer Gassensor bekannt, welcher eine Platinelektrode und eine Goldelektrode aufweist, die mit einem Festelektrolyt kontaktiert sind. Diese sind wiederum mit jeweiligen Leitungen mit einer Erfassungseinrichtung wie beispielsweise einem Millivoltmeter verbunden, um die Messinformation abzugreifen. Der hierin offenbarte Sensor ist insbesondere zur Messung bei verschiedenen Temperaturen ausgelegt, so dass beispielsweise bei einer Temperatur von 300°C eine hohe Empfindlichkeit bei Stickstoffdioxid, bei 400°C eine hohe Empfindlichkeit bei Stickstoffmonoxid und bei 500°C eine hohe Empfindlichkeit bei Kohlenmonoxid vorliegt. Dies zeigt zwar eine Erhöhung der Selektivität durch Veränderung der Temperatur, jedoch neigen die Elektroden aus den Edelmetallen ebenfalls zur Oxidation und können das Messergebnis negativ beeinflussen.

WO 2007/0733111 A1 offenbart einen Gassensor zur Detektion von Gasen mit einer sensitiven Schicht, wobei die sensitive Schicht in einem Elektrospinn-Verfahren erzeugt und auf einem Substrat aufgebracht wird, welches mit einer interdigitalen Struktur vorgesehen ist.

Im Zeitschriftenartikel « A. Helwig et al. "Temperature characterization of silicon substrates for gas sensors by Raman spectroscopy", Sensors and actuators B 126, 240-244, 2007" werden mögliche Aufbau eines Gassensors mit einer lokalen Heizplatte untersucht.

DE 44 01 885 A1 betrifft einen Gasdetektor, welcher ausschließlich aus Materialien besteht, die keine katalytische Aktivitäten entfalten, beispielsweise aus einer Ga2O3-, Sn02-, AIVO4-odcr SrTiO3 -Dünnschicht mit Gold-, Silber-, oder Nickel-Elektroden oder Elektroden aus Legierungen von diesen Metallen (Spalte 4, Zeile 13 bis19).

Folglich entsteht bei derartigen Gassensoren das Problem, dass bei höheren Temperaturen die Metalle in die sensitive Schicht hinein diffundieren und deren gassensitive Eigenschaften beeinflussen. Schon bei niedrigen Temperaturen reagieren die Gase bevorzugt und sehr frühzeitig an den Leiterbahnen und verfälschen die Sensorcharakteristik. Da dünnere Schichten empfindlicher auf die Dotie-. rung reagieren, sind diese noch anfälliger auf die genannte Problematik, sodass das Prinzip der Messung verschiedener Gase mit mikromechanisch aufgebauten Gassensoren noch stärker eingeschränkt ist.

Es ist daher die Aufgabe der vorliegenden Erfindung, einen Gassensor zu schaffen, welcher die Nachteile des vorgenannten Standes der Technik vermeidet und eine Kontaktierung der gassensitiven Schicht aufweist, die die sensitiven Eigenschaften bei der Detektion des Gases durch die Schichten nicht beeinflusst.

Diese Aufgabe wird von einem Gassensor zur Detektion verschiedener Gase gemäß Anspruch 1 sowie einem Verfahren zum Deteletieren verschiedener Gase nach Anspruch 3 gelöst. Vorteilhafte Weiterbildungen der Erfindung sind in den abhängigen Ansprüchen angegeben.

Die Erfindung schließt die technische Lehre ein, dass die Leiterbahn aus einem dotierten Metalloxidmaterial mit nichtkatalytischen Eigenschaften ausgebildet ist, um einen Einfluss durch die Leiterbahn auf die Detektion des Gases zu vermeiden.

Der Gedanke der vorliegenden Erfindung besteht darin, ein Material für die Kontaktierung der gassensitiven Schichten bereitzustellen, welches keine katalytische

Wirkung aufweist und folglich das Messergebnis der Gasdetektion nicht beeinflusst. Hierfür zeigt die Auswahl von dotiertem Metalloxidmaterial überraschend den Vorteil, dass trotz einer hervorragenden elektrischen Leitfähigkeit eine katalytische Wirkung des Materials nicht feststellbar ist und die Sensorschichten nicht durch Platinkontaktierungen katalytisch beeinflusst sind, so dass die Selektivität der gassensitiven Schichten auch bei konstanter Temperatur deutlich erhöht wird.

Vorteilhafterweise umfasst das dotierte Metalloxidmaterial ein mit Antimon dotiertes Zinnoxid (SnO2:Sb). Das Sn02:Sb kann sehr einfach über unterschiedliche Arten hergestellt werden. Beispielsweise bietet sich ein wirtschaftliches Herstellungsverfahren durch ein Elektronenstrahlverdampfen an. Wird das Zinnoxid mit 5 Gew.% dotiert, ist eine hervorragende Leitfähigkeit des dotierten Metalloxidmaterials als Leiterbahn gegeben.

Gemäß einer vorteilhaften Ausführungsform des Gassensors ist der Betrieb der gassensitiven Schicht zur Detektion der Gase nach Art eines resistiven Messverfahrens ausgebildet, bei dem der elektrische Widerstand der gassensitiven Schicht unter Einfluss eines Gases eine Änderung aufweist, über die eine qualitative und/oder quantitative Information über die Art und/oder das Vorhandensein des Gases oder der Gase ermittelbar ist. Der erfindungsgemäße Gassensor mit Leiterbahnen aus dotiertem Metalloxidmaterial kann bevorzugt als beheizter Gassensor gebildet sein, bei dem das Substrat beheizt ist, um die auf diesem aufgebrachte gassensitive Schicht zur Detektion der Gase bei einer Temperatur von 100°C bis 1.100°C, vorzugsweise von 200°C bis 700°C und besonders bevorzugt von 300°C bis 450°C zu betreiben.

Die Leiterbahnen können über bekannte Prozessierungsschritte wie einem Lift-Off-Verfahren einfach strukturiert werden. Nach einem thermischen Annealingschritt ist es voll funktionsfähig. Da es sich um ein Oxid handelt, besteht keine Gefahr der Nachoxidation, so dass eine Stabilität des Sensors erreicht werden kann. Es ist durch die Verwendung eines bereits oxidierten Materials als Leiterbahn hoch temperaturstabil und ist somit für Einsätze bis über 1.000°C geeignet und weist auch bei hohen Temperaturen keine katalytischen Eigenschaften auf. Betreffend des Lift-off-Verfahren zur Aufbringung der Leiterbahnen auf das Substrat erfolgt eine Beschichtung des Substrats mit Fotoresist, welcher nachfolgend an den Stellen, an denen die Leiterbahnbeschichtung wieder entfernt wird, belichtet wird. Dann wird das Substrat flächig mit dem Leiterbahnmaterial, nämlich dem dotierten Metalioxidmaterial, beschichtet. In einem darauf folgenden Ablöseverfahren des Fotoresists von der Oberfläche des Substrates, verbleibt das Metalloxidmaterial nur an den Stellen, an den der Fotoresist nicht belichtet wurde. Im Ergebnis ist eine sehr kleine Struktur erhältlich, welche vorliegend als Leiterbahnstruktur zur Kontaktierung der gassensitiven Schicht Anwendung finden kann. Dieses Lift-Off-Verfahren ist auch für die Aufbringung von SnO2:Sb geeignet.

Die gassensitive Schicht auf dem Substrat kann eine Dicke von ca. 80nm bis 500nm, und vorzugsweise von 100nm aufweisen. Diese Schichtdicke ist lediglich eine nominelle Schichtdicke, wobei auch eine weitere Reduzierung der Schichtdicke bis hin zu einzelnen Nanoröhren oder Nanoclustern im unteren nm-Bereich möglich ist. Das Substrat des Gassensors kann als mikromechanisches Bauteil ausgebildet sein, wobei der Gesamtaufbau des Gassensors in ein Chip-Format überführt werden kann, so dass der Gassensor nach Art eines Lab-on-the-Chip-Sensors ausgeführt sein kann.

Vorzugsweise kann der Gassensor eine gassensitive Schicht aufweisen, die zur Messung von N02 und/oder H2 ausgebildet ist, wobei die Messung von NO2 und H2 entweder bei gleichen oder bei unterschiedlichen Temperaturen erfolgen kann.

Weitere, die Erfindung verbessernde Maßnahmen werden nachstehend gemeinsam mit der Beschreibung eines bevorzugten Ausführungsbeispiels der Erfindung anhand der Figuren näher dargestellt.

Es zeigt:
- Figur 1: eine Leiterbahnstruktur auf einem Substrat, welche aus einem mit Antimon dotierten Zinnoxid ausgebildet ist;
- Figur 2: ein mikromechanisch hergestelltes Gassensorarray mit zwei unterschiedlich sensitiven Schichten;
- Figur 3: beispielhaft einen Response eines reinen Zinnoxid-Sensors für Stickstoffdioxid;
- Figur 4: beispielhaft einen Response des Gassensors mit der gassensitiven Schicht gemäß der Figur 3 für gasförmigen Wasserstoff;
- Figur 5: beispielhaft einen Response eines Gassensors mit einer mit Platin dotierten gassensitiven Zinnoxidschicht für Stickstoffdioxid und
- Figur 6: beispielhaft einen Response eines Gassensors mit einer gassensitiven Schicht gemäß der Figur 5 für gasförmigen Wasserstoff.

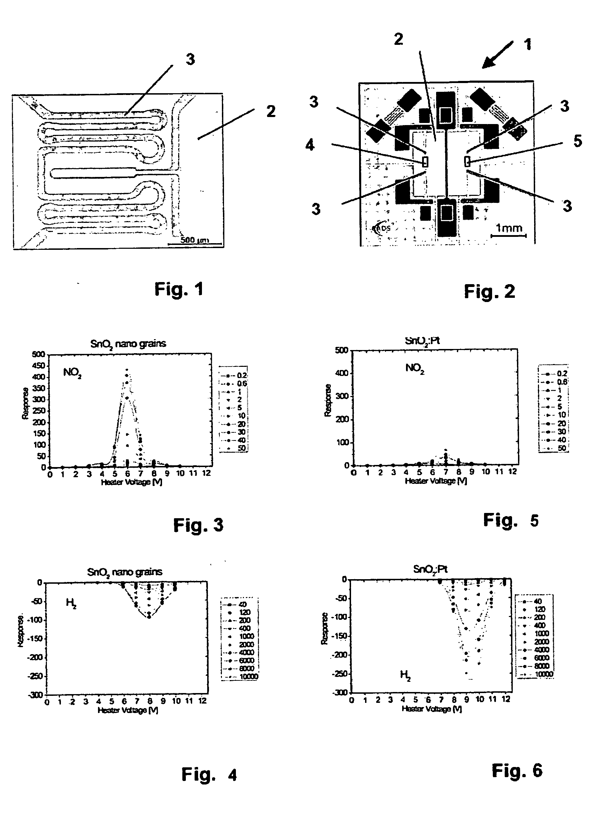

Zur Erläuterung zeigt Figur 1 beispielhaft eine Abbildung einer Leiterbahn 3 auf einem Substrat 2 ohne gassensitive Schichten. Die Leiterbahn 3 weist ein dotiertes Metalloxidmaterial auf, welches aus einem mit Antimon dotierten Zinnoxid (SnO2:Sb) besteht. Die Leiterbahn 3 ist mittels eines lithographischen Lift-Off-Verfahrens aufgebracht worden.

Figur 2 zeigt einen Gassensor 1, weicher als mikromechanisch hergestelltes Gassensorarray ausgeführt ist, das auf der linken Bildseite eine erste gassensitive Schichteinheit 4 und auf der rechten Bildseite eine zweite gassensitive Schichteinheit 5 umfasst. Die jeweiligen Schichteinheiten 4 und 5 sind mit jeweiligen Leiterbahnen 3 kontaktiert, die erfindungsgemäß aus einem mit Antimon dotierten Zinnoxid (SnO2:Sb) bestehen. Die erste gassensitive Schichteinheit 4 umfasst eine sensitive Schicht aus reinem Zinnoxid (SnO2). Diese reine SnO2-Schicht besteht aus Nano-Körnern, wohingegen die rechts abgebildete zweite gassensitive Schichteinheit 5 ebenfalls aus einer Zinnoxid-Schicht (SnO2) besteht, der zusätzlich etwas Platin als Katalysator beigemischt wurde. Mittels dieses Sensorarrays wurden Versuchsergebnisse ermittelt, welche nachfolgend in den Figuren 3 bis 6 beispielhaft dargestellt sind.

In den Figuren 3 und 4 ist in Form eines Diagramms der Gasresponse dargestellt, welcher mit der auf Nano-Körnern basierenden reinen Zinnoxidschicht gemessen wurde. Es ist deutlich erkennbar, dass der Response gemäß der Figur 3 für Stickstoffdioxid deutlich differiert vom Response des Gases Wasserstoff gemäß der Figur 4. Die Versuche wurden bei verschiedenen Temperaturen durchgeführt, wobei unabhängig von der Temperatur das Responseverhalten deutlich voneinander unterscheidbar ist, so dass verschiedene Gase mit einer hohen Selektivität detektierbar sind.

In den Figuren 5 und 6 sind in Diagrammform die Response dargestellt, die mit der zweiten gassensitiven Schichteinheit 5 ermittelt wurden. Trotz der Platin-Dotierung des Zinnoxids ist auch in diesem Fall das Responseverhalten zwischen dem Gas Stickstoffdioxid und dem Gas Wasserstoff deutlich voneinander unterscheidbar. Die Ergebnisse zeigen, dass das Responseverhalten beider Sensoren stark voneinander variiert.

Beide sensitiven Schichten haben ihre selektiven Eigenschaften beibehalten, obwohl eine Schicht mit Platin dotiert wurde. Das Kontaktmaterial der Leiterbahnen 3 (siehe Fig. 1 und 2) zeigt keine katalytischen Eigenschaften und führt somit nicht zu einer Verfälschung des Selektivitätsprofils der einzelnen sensitiven Schichten.

### Bezugszeichenliste

- **1**: Gassensor
- **2**: Substrat
- **3**: Leiterbahn
- **4**: erste gassensitive Schichteinheit
- **5**: zweite gassensitive Schichteinheit

## Patentansprüche

1. Gassensor (1) zur Detektion von Gasen, mit einer ersten gassensitiven Schicht (4) und einer zweiten gassensitiven Schicht (5), die auf einem Substrat (2) aufgebracht sind, wobei weiterhin jeweils eine Leiterbahn (3) zur Kontaktierung der jeweiligen gassensitiven Schichten (4, 5) auf dem Substrat (2) vorgesehen ist, **dadurch gekennzeichnet, dass** die erste gassensitive Schicht (4) aus Nano-Körnern aus reinem Zinnoxid (Sn02) und die zweite gassensitive Schicht (5) aus Zinnoxid mit Platin als Katalysator besteht und wobei die Leiterbahn (3) aus mit Antimon dotierten Zinnoxid (SnO2:Sb) mit nichtkatalytischen Eigenschaften ausgebildet ist, um einen Einfluss durch die Leiterbahn (3) auf die Detektion des Gases zu vermeiden.

2. Gassensor (1) nach Anspruch 1,
**dadurch gekennzeichnet, dass** das Zinkoxid (Sn02) der Leiterbahnen (3) eine Dotierung von 5 Gew.% Antimon (Sb) aufweist.

3. Verfahren zum Detektieren verschiedener Gase mit einem Gassensor (1) nach vorgenannten Ansprüchen,
**dadurch gekennzeichnet, dass** das Substrat (2) des Gassensors (1) beheizt wird, um die auf diesem aufgebrachten gassensitiven Schichten (5, 5) zur Detektion der Gase bei einer Temperatur von 100°C bis 1100°C, vorzugsweise von 200°C bis 700°C und besonders bevorzugt von 300°C bis 450°C zu betreiben.

4. Verfahren zum Detektieren verschiedener Gase nach Anspruch 3,
**dadurch gekennzeichnet, dass** der Betrieb der gassensitiven Schichten (4, 5) zur Detektion der Gase nach Art eines resistiven Messverfahrens ausgebildet ist, bei dem der elektrische Widerstand der gassensitiven Schichten (4, 5) unter Einfluss eines Gases eine Änderung aufweist, über die eine qualitative und/oder quantitative Information über die Art und/oder das Vorhandensein des Gases oder der Gase ermittelbar ist.

5. Verfahren zur Herstellung eines Gassensor (1) nach Anspruch 1 oder 2, **dadurch gekennzeichnet, dass** die Leiterbahnen (3) mittels eines Lift-Off Verfahrens auf das Substrat (2) aufgebracht werden.

## Claims

1. A gas sensor (1) for the detection of gases having a first gas-sensitive layer (4) and a second gas-sensitive layer (5) applied on a substrate (2), wherein furthermore a conductor track (3) is provided on the substrate (2) for contacting the respective gas-sensitive layers (4,5), **characterized in that** the first gas-sensitive layer (4) consists of nano grains made of pure tin oxide (Sn02) and the second gas-sensitive layer (5) consists of tin oxide with platinum as catalyst and wherein the conductor track (3) is made of antimony-doped tin oxide (Sn02:Sb) having non-catalytic properties in order to avoid an influence of the conductor track (3) on the detection of the gas.

2. The gas sensor (1) according to claim 1, **characterized in that** the tin oxide (SnO2( of the conductor tracks (3) comprises a doping with antimony (Sb) of 5 % by weight.

3. A process for detecting various gases with a gas sensor (1) according to any of the proceeding claims, **characterized in that** the substrate (2) of the gas sensor (1) is heated in order to operate the gas-sensitive layers (4,5) for the detection of gases deposited on the substrate at a temperature from 100°C to 1100 °C, preferably from 200 °C to 700°C and especially preferably from 300° C to 450° C.

4. The process for detecting various gases according to claim 3, **characterized in that** the operation of the gas-sensitive layers (4,5) for the detection of gases is of the type of a resistive measuring process, in which the electrical resistance of the gas-sensitive layers (4,5) changes under the influence of a gas, allowing a determination of a qualitative and/or quantitative information on the type and/or the presence of a gas or of gases.

5. The process for manufacturing a gas sensor (1) according to claim 1 or 2, **characterized in that** the conductor tracks (3) are deposited on the substrate (2) via a lift-off process

## Revendications

1. Un capteur à gaz (1) pour la détection de gaz pourvu d'une première couche sensible au gaz (4) et d'une seconde couche sensible au gaz (5) qui sont disposées sur un substrat (2) et, en outre, présentent chacune une piste conductrice (3) pour l'établissement du contact entre les couches sensibles au gaz (4, 5) sur le substrat (2),
**caractérisé en ce que** la première couche sensible au gaz (4) se compose de nanograins d'oxyde d'étain (Sn02) et la seconde couche sensible au gaz (5) d'oxyde d'étain et de platine en tant que catalyseur, et **en ce que** la piste conductrice (3) se compose d'oxyde d'étain pourvu d'antimoine (Sn02 :Sb) présentant des propriétés non-catalytiques afin d'éviter toute influence de la piste conductrice (3) sur la détection des gaz.

2. Capteur à gaz (1) selon la Revendication 1,
**caractérisé en ce que** l'oxyde d'étain (Sn02) de la piste conductrice (3) se compose de 5%poids d'antimoine (Sb).

3. Procédé pour la détection de divers gaz au moyen d'un capteur à gaz (1) selon les Revendications précédentes,
**caractérisé en ce que** le substrat (2) du capteur à gaz (1) est chauffé de sorte que les couches sensibles au gaz (4, 5) disposées sur le substrat et destinées à la détection des gaz soient exploitées à une température comprise entre 100°C et 1100°C, de préférence entre 200°C et 700°C et idéalement entre 300°C et 450°C.

4. Procédé pour la détection de divers gaz selon la Revendication 4,
**caractérisé en ce que** l'exploitation des couches sensibles au gaz (4, 5) pour la détection des gaz est réalisée conformément à un procédé de mesure de la résistance selon lequel la résistance électrique des couches sensibles aux gaz (4, 5) présente une modification sous l'influence d'un gaz au moyen de laquelle il est possible de déterminer une information qualitative et/ou quantitative sur le type et/ou la présence d'un ou de plusieurs gaz.

5. Procédé pour la fabrication d'un capteur de gaz (1) selon la Revendication 1 ou 2,
**caractérisé en ce que** la piste conductrice (3) est disposée sur le substrat (2) au moyen d'un procédé de lift-off.
